(19) Europäisches Patentamt
European Patent Office
Office européen des brevets

(11) Veröffentlichungsnummer: **0 277 538 A2**

(12) **EUROPÄISCHE PATENTANMELDUNG**

(21) Anmeldenummer: **88100648.0**

(51) Int. Cl.[4]: **C07D 213/64** , A01N 43/40

(22) Anmeldetag: **19.01.88**

(30) Priorität: **31.01.87 DE 3702922**

(43) Veröffentlichungstag der Anmeldung: **10.08.88 Patentblatt 88/32**

(84) Benannte Vertragsstaaten: **BE CH DE FR GB IT LI NL**

(71) Anmelder: **BAYER AG**
**D-5090 Leverkusen 1, Bayerwerk(DE)**

(72) Erfinder: **Förster, Heinz, Dr.**
**Am Eckbusch 47**
**D-5600 Wuppertal 1(DE)**
Erfinder: **Riebel, Hans-Jochem, Dr.**
**In der Beek 92**
**D-5600 Wuppertal 1(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Strang, Robert Harry, Dr.**
**Unterdorfstrasse 6A**
**D-4000 Düsseldorf 31(DE)**

(54) **Optisch aktiver 2-(4-(3,5-Dichlor-2-pyridinyl-oxy)-phenoxy)-propionsäure-(3-benzyloxy-propyl)-ester, Verfahren zu dessen Herstellung und dessen Verwendung als Herbizid.**

(57) Rechtsdrehender 2-(4-(3,5-Dichlor-2-pyridinyl-oxy)-phenoxy)-propionsäure-(3-benzyloxy-propyl)-ester der Formel

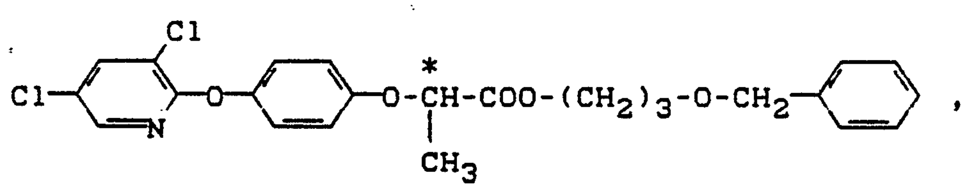

Verfahren zu seiner Herstellung und seine Verwendung als Herbizid.

EP 0 277 538 A2

**Optisch aktiver 2-(4-(3,5-Dichlor-2-pyridinyl-oxy)-phenoxy)-propionsäure-(3-benzyloxy-propyl)-ester, Verfahren zu dessen Herstellung und dessen Verwendung als Herbizid**

Die Erfindung betrifft den neuen rechtsdrehenden*) 2-(4-(3,5-Dichlor-2-pyridinyl-oxy)-phenoxy)-propionsäure-(3-benzyloxy-propyl)-ester, Verfahren zu dessen Herstellung und dessen Verwendung als Herbizid.

*) Unter rechtsdrehenden Verbindungen (rechtsdrehenden Enantiomeren) sind die optisch aktiven Verbindungen zu verstehen, welche die Schwingungsebene von linear polarisiertem Licht nach rechts drehen.

Es ist bereits bekannt, daß bestimmte Phenoxy-propionsäure-Derivate herbizide Eigenschaften besitzen (vgl. EP-A 68 260). So kann zum Beispiel racemischer 2-(4-(3,5-Dichlor-2-pyridinyl-oxy)-phenoxy)-propionsäure-(2-benzyloxy-ethyl)-ester zur Bekämpfung von Unkraut eingesetzt werden. Die Wirkung dieses Stoffes ist jedoch nicht immer ausreichend.

Es wurde nun der neue rechtsdrehende 2-(4-(3,5-Dichlor-2-pyridinyl-oxy)-phenoxy)-propionsäure-(3-benzyloxy-propyl)-ester der Formel (I)

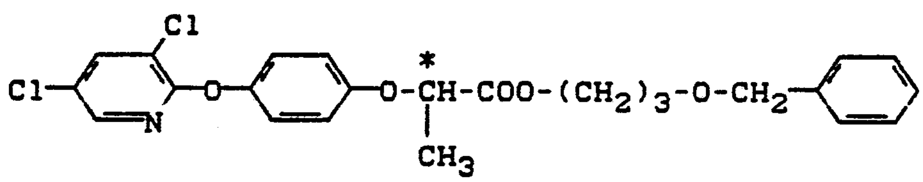

(I)

gefunden.

Weiter wurde gefunden, daß man den neuen rechtsdrehenden 2-(4-(3,5-Dichlor-2-pyridinyl-oxy)-phenoxy)-propionsäure-(3-benzyloxy-propyl)-ester der Formel (I) erhält, wenn man

(a) rechtsdrehendes 2-(4-(3,5-Dichlor-2-pyridinyl-oxy)-phenoxy)-propionsäure-chlorid der Formel (II)

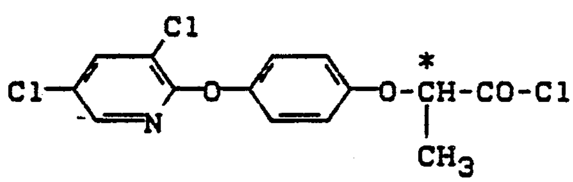

(II)

mit 3-Benzyloxy-propanol der Formel (III)

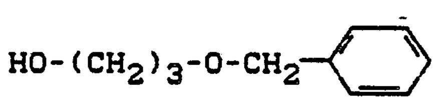

(III)

gegebenenfalls in Gegenwart eines Säureakzeptors und gegebenenfalls in Gegenwart eines Verdünnungsmittels umsetzt, oder

(b) 4-(3,5-Dichlor-2-pyridinyl-oxy)-phenol der Formel (IV)

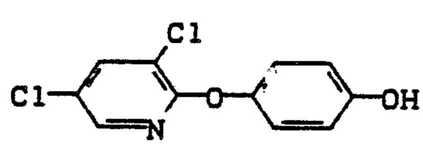

(IV)

mit einem linksdrehenden - bzw. an dem mit einem * markierten Kohlenstoffatom entgegengesetzt zu (I) konfiguriertem - Sulfonyloxy-propionsäure-(3-benzyloxy-propyl)-ester der Formel (V)

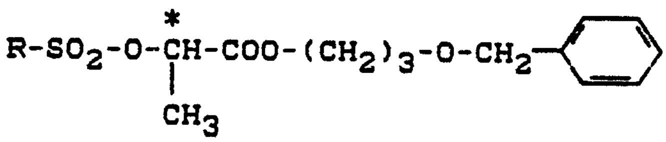

(V)

in welcher
R für gegebenenfalls durch Halogen substituiertes Alkyl oder für gegebenenfalls durch Halogen oder Alkyl substituiertes Aryl steht,
gegebenenfalls in Gegenwart eines Säureakzeptors und gegebenenfalls in Gegenwart eines Verdünnungsmittels umsetzt.

Schließlich wurde gefunden, daß der neue rechtsdrehende 2-(4-(3,5-Dichlor-2-pyridinyl-oxy)-phenoxy)-propionsäure-(3-benzyloxy-propyl)-ester der Formel (I) hervorragende herbizide Wirksamkeit zeigt.

Überraschenderweise besitzt der erfindungsgemäße rechtsdrehende 2-(4-(3,5-Dichlor-2-pyridinyl-oxy)-phenoxy)-propionsäure-(3-benzyloxy-propyl)ester der Formel (I) wesentlich bessere herbizide Eigenschaften als der bekannte racemische 2-(4-(3,5-Dichlor-2-pyridinyl-oxy)-phenoxy)-propionsäure-(2-benzyloxy-ethyl)-ester, welcher ein chemisch ähnlicher Stoff analoger Wirkungsart ist.

Der Verlauf des erfindungsgemäßen Verfahrens (a) kann durch das folgende Formelschema wiedergegeben werden:

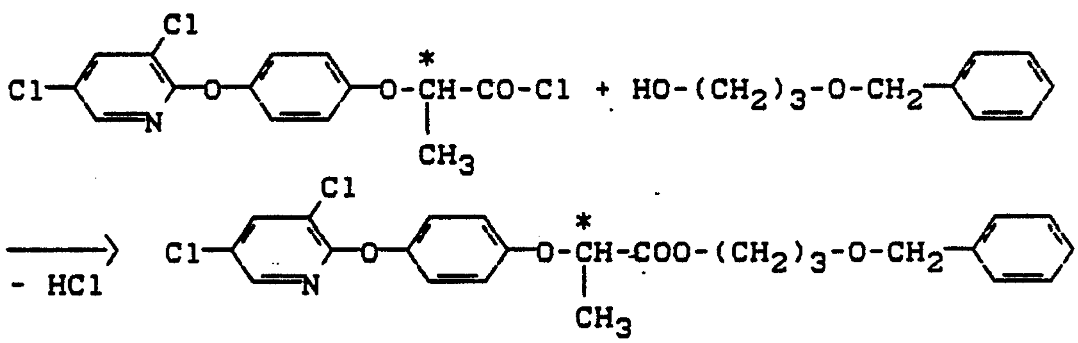


Der Verlauf des erfindungsgemäßen Verfahrens (b) kann - bei Verwendung von 2-Mesyloxy-propionsäure-(3-benzyloxy-propyl)-ester - durch das folgende Formelschema wiedergegeben werden:

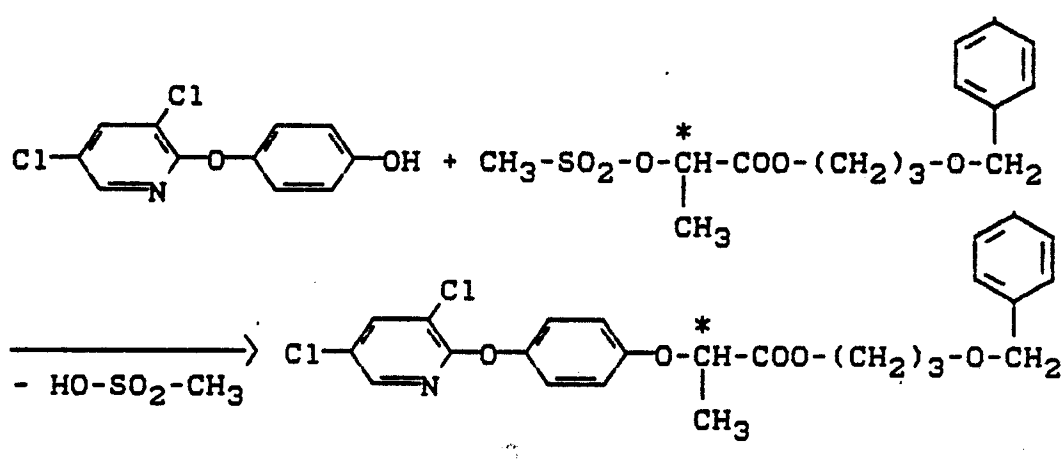


Das beim erfindungsgemäßen Verfahren (a) als Ausgangsstoff zu verwendende rechsdrehende 2-(4-(3,5-Dichlor-2-pyridinyl-oxy)-phenoxy)-propionsäure-chlorid der Formel (II) ist bereits bekannt (vgl. DE-OS 34 02 982). Es kann durch Umsetzung von rechtsdrehender 2-(4-(3,5-Dichlor-2-pyridinyl-oxy)-phenoxy)-propionsäure mit Thionylchlorid hergestellt werden (vgl. DE-OS 27 58 002).

Der beim erfindungsgemäßen Verfahren (a) weiter als Ausgangsstoff zu verwendende 3-Benzyloxy-propanol der Formel (III) ist ebenfalls bereits bekannt (vgl. EP-A 68 260).

Das beim erfindungsgemäßen Verfahren (b) als Ausgangsstoff zu verwendende 4-(3,5-Dichlor-2-pyridinyl-oxy)-phenol der Formel (IV) ist bereits bekannt (vgl. DE-OS 25 46 251).

Die beim erfindungsgemäßen Verfahren (b) weiter als Ausgangsstoffe zu verwendenden linksdrehenden Sulfonyloxy-propionsäure-(3-benzyloxy-propyl)-ester sind durch die Formel (V) allgemein definiert. In dieser Formel steht R vorzugsweise für gegebenenfalls durch Fluor substituiertes $C_1$-$C_8$-Alkyl oder für gegebenenfalls durch Fluor, Chlor, Brom oder $C_1$-$C_4$-Alkyl substituiertes Phenyl.

Als Beispiele für die Verbindungen der Formel (V) seien genannt:
2-Methylsulfonyloxy-, 2-Ethylsulfonyloxy-, 2-Trifluormethylsulfonyloxy-, 2-Phenylsulfonyloxy-, 2-(4-Methylphenyl)-sulfonyloxy-, 2-(4-Fluor-phenyl)-sulfonyloxy-, 2-(4-Chlor-phenyl)-sulfonyloxy-und 2-(4-Brom-phenyl)-sulfonyloxy-propionsäure-(3-benzyloxy-propyl)-ester.

Dier Verbindungen der Formel (V) sind bekannt und/oder können nach an sich bekannten Verfahren hergestellt werden (vgl. EP-A 112 531).

Die erfindungsgemäßen Verfahren (a) und (b) werden vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Me thyl-isopropyl-und Methyl-isobutyl-keton, Ester wie Essigsäuremethylester undethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise in Frage kommen Alkalimetallhydroxide wie. z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäße Verfahren (a) und (b) innerhalb eines größeren Bereiches variiert werden. Im allgemeinen arbeitet man bei Temperaturen zwischen -20 °C und +160 °C, vorzugsweise bei Temperaturen zwischen -10 °C und +100 °C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren. Im allgemeinen geht man so vor, daß man das Reaktionsgemisch - gegebenenfalls nach Einengen und Aufnehmen des Rückstandes in einem mit Wasser praktisch nicht mischbaren Lösungsmittel, wie z. B. Toluol oder Methylenchlorid - mit einer verdünnten Mineralsäure, wie z. B. Salzsäure, dann mit einer wässrigen Base, wie z. B. Sodalösung, un anschließend mit Wasser wäscht, mit einem üblichen Trockenmittel, wie z. B. Magnesiumsulfat, trocknet und filtriert. Nach sorgfältigem Abdestillieren des Lösungsmittels vom Filtrat erhält man einen Rückstand, welcher im wesentlichen des gewünschte Produkt der Formel (I) enthält.

Der erfindungsgemäße Wirkstoff der Formel (I) kann als Defoliant, Desiccant, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob der erfindungsgemäße Stoff als totales oder selektives Herbizid wirkt, hängt im wesentlichen von der angewendeten Menge ab.

Der erfindungsgemäße Wirkstoff kann z. B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung des erfindungsgemäßen Wirkstoffes ist jedoch keineswegs auf diese Gattungen beschränkt, sondern erstreckt sich in gleicher Weise auch auf andere Pflanzen.

Der erfindungsgemäße Wirkstoff der Formel (I) eignet sich vor allem zur selektiven Bekämpfung von monokotylen Unkräutern in monokotylen und dikotylen Kulturen, insbesondere im Nachauflauf-Verfahren.

Der erfindungsgemäße Wirkstoff kann in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann als solcher oder in seinen Formulierungen auch in Mischung mit bekannten Herbiziden zur Umkrautbekämpfung Verwendung finden, wobei Fertigformulierungen oder

Tankmischungen möglich sind.

Für die Mischungen kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, N,N-Dimethyl-N'-(3-chlor-4-methylphenyl)-harnstoff; N,N-Dimethyl-N'-(4-isopropylphenyl)-harnstoff; 4-Ethylamino-2-t-butylamino-6-methylthio-s-triazin; 4-Amino-6-t-butyl-3-ethylthio-1,2,4-triazin-5(4H)-on; 2-Chlor-N-{[(4-methoxy-6-methyl-1,3,5-triazin-2-yl)-amino]-carbonyl}-benzolsulfonamid; 2-{[[((4-Methoxy-6-methyl-1,3,5-triazin-2-yl)-amino)-carbonyl]-amino]-sulfonyl}-benzoesäure oder deren Methylester; 5-(2-Chlor-4-trifluormethyl-phenoxy)-N-methylsulfonyl-2-nitrobenzamid; 5-(2-Chlor-4-trifluormethyl-phenoxy)-2-nitro-benzoesäure; 3,5-Diiod-4-hydroxybenzonitril; 3,5-Dibrom-4-hydroxy-benzonitril; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 2-[5-Methyl-5-(1-methylethyl)-4-oxo-2-imidazolin-2-yl]-3-chinolincarbonsäure; 3-(Ethoxycarbonylaminophenyl)-N-(3'-methylphenyl)-carbamat; 0-(6-Chlor-3-phenyl-pyridazin-4-yl)-S-octyl-thiocarbonat, 3-Isopropyl-2,1,3-benzothiadiazin-4-on-2,2-dioxid und (2-Ethoxy-1-methyl-2-oxo-ethyl)-5-[2-chlor-4-(trifluormethyl)-phenoxyl]-2-nitrobenzoat in Frage. Einige Mischungen zeigen auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Der erfindungsgemäße Wirkstoff kann als solcher, in Form seiner Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Der erfindungsgemäße Wirkstoff kann sowohl vor als auch nach dem Auflaufen der Pflanzen appliziert werden.

Er kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung des erfindungsgemäßen Wirkstoffes geht aus den nachfolgenden Beispielen hervor.

Herstellungsbeispiel

Beispiel 1

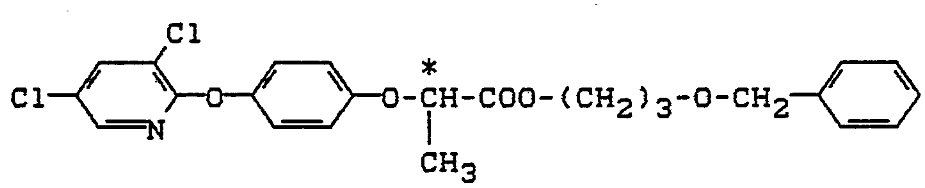

Eine Lösung von 88,65 g (0,246 Mol) rechtsdrehendem 2-(4-(3,5-Dichlor-2-pyridinyl-oxy)-phenoxy)-propionsäurechlorid in 200 ml Toluol wird bei 20 °C unter Rühren zu einer Lösung aus 43,7, g (0,246 Mol) 3-Benzyloxy-propanol und 24,6 g (0,31 Mol) Pyridin in 800 ml Toluol tropfenweise gegeben. Das Reaktionsgemisch wird 12 Stunden bei 20 °C gerührt, dann mit 200 ml Toluol verdünnt und mit 500 ml 2N-Salzsäure und dann mit 500 ml einer gesättigten Sodalösung gewaschen. Nach dem Neutralwaschen der organischen Phase mit Wasser und dem Trocknen über Magnesiumsulfat wird das Toluol im Vakuum abdestilliert.

Man erhält 110,5 g (96 % der Theorie) rechtsdrehenden 2-(4-(3,5-Dichlor-2-pyridinyl-oxy)-phenoxy-propionsäure-(3-benzyloxy-propyl)-ester als hellbraunes Öl.

Drehwert:

(1-molare Lösung in Chloroform;
Küvettenlänge = 10 cm
R : S-Verhältnis: 87 : 13

Anwendungsbeispiele

In den folgenden Anwendungsbeispielen wird die nachstehend aufgeführte Verbindung als Vergleichssubstanz herangezogen:

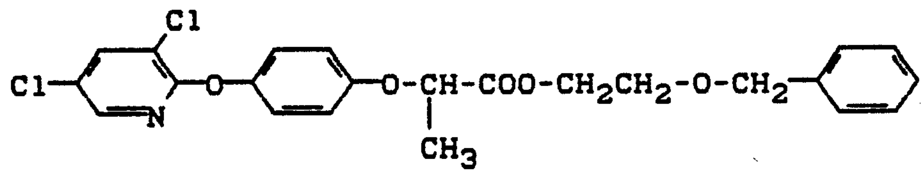

(A)

2-(4-(3,5-Dichlor-2-pyridinyl-oxy)-phenoxy)-propionsäure-(2-benzyloxy-ethyl)-ester (Racemat)
(bekannt aus EP-A 68 260)

Beispiel A

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschte Wirkstoffmenge pro Flächeneinheit ausgebracht werden. Die Konzentration der Spritzbrühe wird so gewählt, daß in 2000 l Wasser/ha die jeweils gewünschte Wirkstoffmenge ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt der Wirkstoff gemäß dem Herstellungsbeispiel (1) bei der Bekämpfung von Unkräutern, wie z. B. Alopecurus, Digitaria, Poa und Sorghum stärkere Wirkung als die Vergleichssubstanz (A).

## Tabelle A

Post-emergence-Test / Gewächshaus

| Wirkstoffe | Aufwandmenge (g/ha) | Zucker-rüben | Soja | Weizen | Alope-curus | Digi-taria | Poa | Sorg-hum |
|---|---|---|---|---|---|---|---|---|
| (A) | 60 | 0 | 0 | 0 | 0 | 90 | 40 | 80 |
| (1) | 60 | 0 | 0 | 0 | 80 | 100 | 85 | 100 |

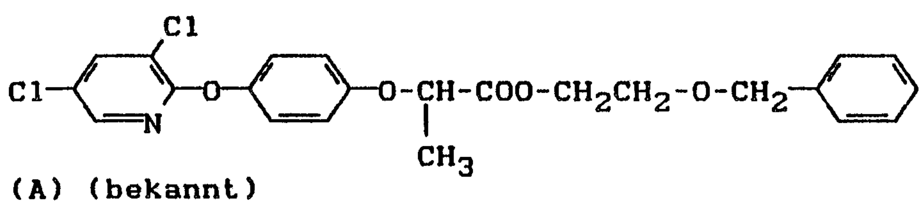

(A) (bekannt)

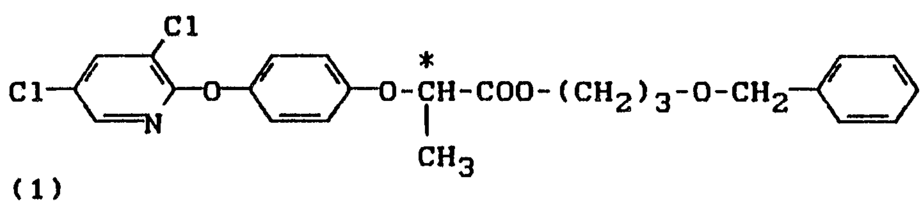

(1)

**Ansprüche**

1. Rechtsdrehender 2-(4-(3,5-Dichlor-2-pyridinyl-oxy)-phenoxy)-propionsäure-(3-benzyloxy-propyl)-ester der Formel (I)

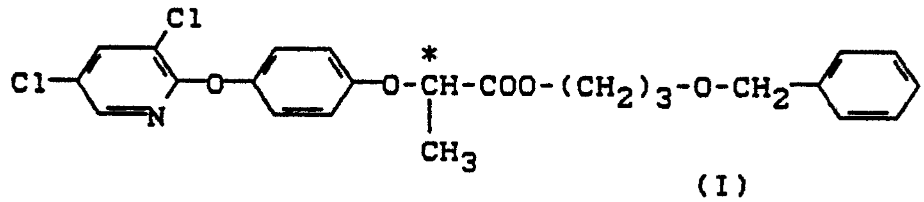

(I)

2. Verfahren zur Herstellung des rechtsdrehenden 2-(4-(3,5-Dichlor-2-pyridinyl-oxy)-phenoxy)-propionsäure-(3-benzyloxy-propyl)-ester der Formel (I)

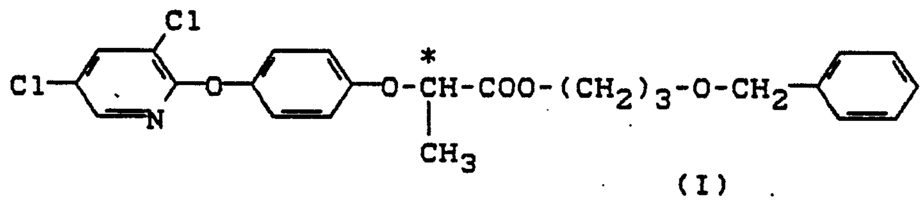

(I) .

dadurch gekennzeichnet, daß man
(a) rechtsdrehendes 2-(4-(3,5,-Dichlor-2-pyridinyl-oxy)-phenoxy)-propionsäure-chlorid der Formel (II)

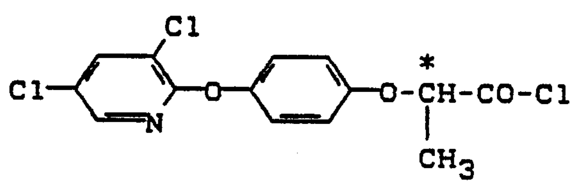

(II)

mit 3-Benzyloxy-propanol der Formel (III)

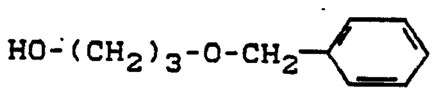

(III)

gegebenenfalls in Gegenwart eines Säureakzeptors und gegebenenfalls in Gegenwart eines Verdünnungsmittels umsetzt, oder
(b) 4-(3,5-Dichlor-2-pyridinyl-oxy)-phenol der Formel (IV)

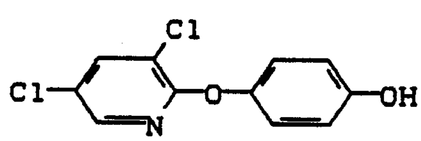

(IV)

mit einem linksdrehenden - bzw. an dem mit einem * markierten Kohlenstoffatom entgegengesetzt zu (I) konfiguriertem -Sulfonyloxy-propionsäure-(3-benzyloxy-propyl)-ester der Formel (V)

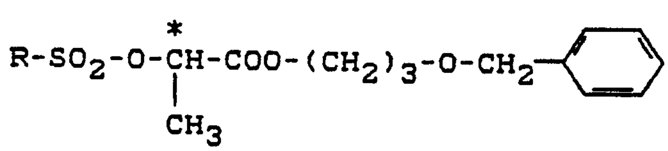

(V)

in welcher
R für gegebenenfalls durch Halogen substituiertes Alkyl oder für gegebenenfalls durch Halogen oder Alkyl substituiertes Aryl steht,
gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an rechtsdrehendem 2-(4-(3,5-Dichlor-2-pyridinyl-oxy)-phenoxy)-propionsäure-(3-benzyloxypropyl)-ester der Formel (I) gemäß Anspruch 1 und 2.

4. Verfahren zur Bekämpfung von Unkräutern, dadurch gekennzeichnet, daß man rechtsdrehenden 2-(4-(3,5-Dichlor-2-pyridinyl-oxy)-phenoxy)-propionsäure-(3-benzyloxypropyl)-ester der Formel (I) gemäß Anspruch 1 und 2 auf die Unkräuter und/oder ihren Lebensraum einwirken läßt.

5. Verwendung von rechtsdrehendem 2-(4-(3,5-Dichlor-2-pyridinyl-oxy)-phenoxy)-propionsäure-(3-benzyloxypropyl)-ester der Formel (I) gemäß Anspruch 1 und 2 zur Bekämpfung von Unkräutern.

6. Verfahren zur Herstellung von herbiziden Mitteln, dadurch gekennzeichnet, daß man rechsdrehenden 2-(4-(3,5-Dichlor-2-pyridinyl-oxy)-phenoxy)-propionsäure-(3-benzyloxypropyl)-ester der Formel (I) gemäß Anspruch 1 und 2 mit Streckmitteln und/oder oberflächenaktiven Substanzen vermischt.